# EUROPEAN PATENT APPLICATION

(11) **EP 2 258 393 A1**
(43) Date of publication of application: **08.12.2010**
(21) Application number: 09727773.5
(22) Date of filing: 28.01.2009
(51) Int. Cl.: A61K 45/00, A61K 48/00, A61P 35/00, A61P 43/00

(54) **ABC TRANSPORTER PROTEIN EXPRESSION INHIBITOR**

(30) Priority: 31.03.2008 JP 2008090729
(71) Applicant: Sugimoto, Yoshikazu, Kashiwa-shi, Chiba 277-0061 (JP)
(72) Inventor: Sugimoto, Yoshikazu, Kashiwa-shi, Chiba 277-0061 (JP)
(74) Representative: Blodig, Wolfgang
(86) International application number: PCT/JP2009/000320
(87) International publication number: WO 2009/122639

(57) **Abstract**

Provided is a novel drug which exhibits excellent effect of inhibiting expression of an ABC transporter protein, as well as improved safety.

An ABC transporter protein expression inhibitor containing, as an active ingredient, a substance which inhibits expression of one or more genes selected from the group consisting of ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, and ZNF259.

## Description

### Technical Field

The present invention relates to an ABC transporter protein expression inhibitor.

### Background Art

Anticancer drugs such as camptothecins (e.g., irinotecan hydrochloride) and mitoxantrone exhibit considerably excellent effect against malignant tumors, and thus have been widely used in clinical settings. However, researchers have pointed out that prolonged and continuous use of such an agent may result in reduction in anticancer effect. Recent research on the mechanism by which cancer cells acquire resistance to such an anticancer drug has shown that BCRP, which is an ABC transporter, participates in the acquisition of anticancer drug resistance (Non-Patent Document 1). Specifically, according to the findings of the research, after prolonged continuous use of such an anticancer drug, BCRP comes to be expressed in cancer cells, and the BCRP discharges the anticancer drug out of the cells to thereby reduce the amount of the anticancer drug accumulated within the cells. In this connection, P-glycoprotein encoded by MDR1 gene is also known as an ABC transporter which participates in the acquisition of anticancer drug resistance (Non-Patent Document 2). P-glycoprotein has two ATP-binding cassettes and exhibits substrate specificity different from that of BCRP.

Hitherto reported ABC transporter inhibitors include a drug-resistance-overcoming agent containing a diphenylacetylpiperazine derivative as an active ingredient (Patent Document 1), an ABC transporter inhibitor containing an enniatin compound as an active ingredient (Patent Document 2), and a P-glycoprotein inhibitor containing an anthranilic acid derivative as an active ingredient (Patent Document 3). However, each of these agents fails to sufficiently exhibit an effect required of an ABC transporter inhibitor, and poses a problem in that it may cause undesirable side effects, due to its low specificity to the protein to be inhibited.

### Related Art Document

Patent Document 1: JP-A-2004-339073
Patent Document 2: JP-A-2005-247716
Patent Document 3: JP-A-2001-502683

Non-Patent Document 1: Proc. Natl. Acad. Sci. USA, 95 (26), 15665-15670 (1998)
Non-Patent Document 2: Methods in Enzymology, 292: 248-594 (1998)

### Disclosure of the Invention

### Problems to be Solved by the Invention

In view of the foregoing, an object of the present invention is to provide a novel drug which exhibits excellent effect of inhibiting expression of an ABC transporter protein, as well as improved safety.
Another object of the present invention is to provide a screening method for selecting an ABC transporter protein expression inhibitor.
Yet another object of the present invention is to provide a method for determining sensitivity to an anticancer drug capable of serving as a substrate for an ABC transporter protein, a method for predicting the degree of side effects which may occur after administration of an anticancer drug capable of serving as a substrate for an ABC transporter protein, and a method for determining anticancer drug resistance by the mediation of an ABC transporter protein. Means for Solving the Problems

In order to achieve the aforementioned objects, the present inventor has carried out screening of a variety of substances with an aim to identify a compound capable of inhibiting expression of an ABC transporter protein by use of human breast cancer cells expressing exogenous P-glycoprotein (MCF-7/MDR) and human breast cancer cells expressing exogenous BCRP (MCF-7/BCRP), and has found that siRNA targeting the following gene; i.e., ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, or ZNF259, inhibits expression of P-glycoprotein in MCF-7/MDR cells and expression of BCRP in MCF-7/BCRP cells.

Accordingly, the present invention provides an ABC transporter protein expression inhibitor containing, as an active ingredient, a substance which inhibits expression of one or more genes selected from the group consisting of ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, and ZNF259.

The present invention also provides an agent for overcoming anticancer drug resistance (hereinafter may be referred to as an "anticancer-drug-resistance-overcoming agent") for cancer cells which have acquired anticancer drug resistance by the mediation of an ABC transporter protein, the agent containing the aforementioned substance as an active ingredient.

The present invention also provides a pharmaceutical composition comprising the aforementioned substance in combination with an anticancer drug capable of serving as a substrate for an ABC transporter protein.

The present invention also provides a screening method for selecting an ABC transporter protein expression inhibitor, comprising searching a substance which inhibits expression of one or more genes selected from the group consisting of ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, and ZNF259.

The present invention also provides a method for determining sensitivity to an anticancer drug capable of serving as a substrate for an ABC transporter protein, comprising measuring the expression level of one or more genes selected from the group consisting of ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, and ZNF259.

The present invention also provides a method for predicting the degree of side effects which may occur after administration of an anticancer drug capable of serving as a substrate for an ABC transporter protein, comprising measuring the expression level of any of the aforementioned genes.

The present invention also provides a method for determining anticancer drug resistance by the mediation of an ABC transporter protein, comprising measuring the expression level of any of the aforementioned genes.

The present invention also provides use of the aforementioned substance for producing an ABC transporter protein expression inhibitor, or an anticancer-drug-resistance-overcoming agent for cancer cells which have acquired anticancer drug resistance by the mediation of an ABC transporter protein.
The present invention also provides use, for producing a pharmaceutical composition, of the aforementioned substance and an anticancer drug in combination, wherein the anticancer drug is capable of serving as a substrate for an ABC transporter protein.
The present invention also provides a method for inhibiting expression of an ABC transporter protein, or a method for overcoming the anticancer drug resistance of cancer cells that has been acquired by the mediation of an ABC transporter protein, comprising administering an effective amount of the aforementioned substance to a subject in need thereof.
The present invention also provides a method for treating cancer, comprising administering, to a subject in need thereof, the aforementioned substance and an anticancer drug capable of serving as a substrate for an ABC transporter protein.

### Effects of the Invention

The present invention realizes recovery of the efficacy of an anticancer drug which has failed to exhibit sufficient efficacy due to expression of an ABC transporter protein (in particular, BCRP or P-glycoprotein). Therefore, the present invention facilitates control of the dose of the anticancer drug, and thus realizes a cancer chemotherapy with reduced side effects.
Also, the present invention realizes provision of a useful drug development system for searching a substance which inhibits expression of an ABC transporter protein (in particular, BCRP or P-glycoprotein), or for elucidating the mechanism of action of the substance.
In addition, according to the present invention, measurement of the expression level of a gene of interest realizes prediction of the degree of side effects which may occur after administration of an anticancer drug, or the degree of anticancer drug resistance. Therefore, the present invention is effective for establishing a safer guideline for anticancer drug therapy.

### Best Modes for Carrying Out the Invention

In the ABC transporter protein expression inhibitor of the present invention, the gene of interest is one or more genes selected from the group consisting of ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, and ZNF259.
As used herein, "ARHGAP17" refers to a gene having the nucleotide sequence represented by GenBank Accession No. NM_018054, or a homologue thereof;
"CTDSP2" refers to a gene having the nucleotide sequence represented by GenBank Accession No. NM_005730, or a homologue thereof;
"DUSP1" refers to a gene having the nucleotide sequence represented by GenBank Accession No. NM_004417, or a homologue thereof;
"IMPA2" refers to a gene having the nucleotide sequence represented by GenBank Accession No. NM_014214, or a homologue thereof;
"RHOBTB3" refers to a gene having the nucleotide sequence represented by GenBank Accession No. NM_014899, or a homologue thereof;
"SGK" refers to a gene having the nucleotide sequence represented by GenBank Accession No. NM_005627, or a homologue thereof;
"UBE2H" refers to a gene having the nucleotide sequence represented by GenBank Accession No. NM_003344, or a homologue thereof;
"INPP5F" refers to a gene having the nucleotide sequence represented by GenBank Accession No. NM_014937, or a homologue thereof;
"MAP2K6" refers to a gene having the nucleotide sequence represented by GenBank Accession No. NM_002758, or a homologue thereof;
"PPM1E" refers to a gene having the nucleotide sequence represented by GenBank Accession No. NM_014906, or a homologue thereof;
"PRKAG2" refers to a gene having the nucleotide sequence represented by GenBank Accession No. NM_016203, or a homologue thereof;
"PRKCD" refers to a gene having the nucleotide sequence represented by GenBank Accession No. NM_006254, or a homologue thereof;
"PTPN21" refers to a gene having the nucleotide sequence represented by GenBank Accession No. NM_007039, or a homologue thereof;
"UBE2B" refers to a gene having the nucleotide sequence represented by GenBank Accession No. NM_003337, or a homologue thereof;
"UBTF" refers to a gene having the nucleotide sequence represented by GenBank Accession No. NM_014233, or a homologue thereof; and
"ZNF259" refers to a gene having the nucleotide sequence represented by GenBank Accession No. NM_003904, or a homologue thereof.

In the present invention, no particular limitation is imposed on the type of the ABC transporter whose expression is to be inhibited. Examples of the ABC transporter which has been reported to be involved in drug resistance include P-glycoprotein, BCRP, MRP1, MRP2, MRP3, MRP4, MRP5, MRP7, ABCA/ABC2, and ABCB5. Of these, P-glycoprotein and BCRP are associated with resistance to a variety of anticancer drugs, and thus are particularly important target proteins of the inhibitor of the present invention.

Expression of such an ABC transporter protein is inhibited by inhibiting expression of a gene of interest in the present invention; i.e., ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, or ZNF259.

In the present invention, no particular limitation is imposed on the "substance which inhibits expression of a gene of interest," but the substance employed is preferably, for example, siRNA, sense RNA, antisense RNA, shRNA, ribozyme, DNAzyme, oligonucleotide, monoclonal antibody, polyclonal antibody, or miRNA. Of these, siRNA is particularly preferably employed, from the viewpoints of high specificity to a target gene, as well as no induction of undesirable side effects.
Inhibition of expression of a gene of interest in the present invention may be carried out by use of any of the aforementioned substances, or may be carried out through replacement (knockout) of an endogenous gene or a similar technique. Inhibition of expression of an ABC transporter protein may be carried out by use of, for example, a known inhibitor against a protein encoded by a gene of interest.

The siRNA employed in the present invention may be produced through artificially synthesizing, by means of an RNA synthesizer, an RNA fragment corresponding to the nucleotide sequence of the sense strand of a gene of interest whose expression is to be inhibited or a portion of the sense strand, and an RNA fragment complementary to the aforementioned RNA fragment.
Alternatively, the siRNA employed in the present invention may be produced through the following procedure: an expression vector (e.g., virus vector or plasmid) in which a gene of interest whose expression is to be inhibited or a portion thereof has been cloned in the forward and reverse directions is incorporated into cells, and both strands of DNA are expressed in the cells so that siRNA is produced in the cells and target mRNA is degraded.
The siRNA employed in the present invention may be produced by use of an expression vector incorporating a DNA fragment having a sequence formed through fusion of the sequences of both strands of a DNA fragment corresponding to a gene of interest whose expression is to be inhibited or a portion thereof; specifically, a DNA fragment having a sequence formed through fusion of the 3' end of the sense strand of the DNA fragment with the 5' end of the antisense strand thereof, or a DNA fragment having a sequence formed through fusion of the 3' end of a DNA fragment complementary to the DNA fragment with the 5' end of the sense strand of the DNA fragment. The aforementioned virus vector may be, for example, an adenovirus vector or a retrovirus vector. The siRNA employed in the present invention preferably includes 30 or less nucleotides, more preferably 21 to 23 nucleotides, particularly preferably 21 nucleotides.

No particular limitation is imposed on the siRNA employed in the present invention, so long as it is formed of an RNA fragment corresponding to the nucleotide sequence of the sense strand of a gene of interest whose expression is to be inhibited or a portion of the sense strand, and an RNA fragment complementary to the aforementioned RNA fragment. For example, siRNAs shown in Tables 1 to 16 are preferably employed. The target sequences of these siRNAs are represented by SEQ ID NOs: 1 to 16. These siRNAs are commercially available from Qiagen.

**[Table 1]**

| |
|---|
| Cat. no.: SI02780449 |
| Product Name: Hs_ARHGAP17_5 HP GenomeWide siRNA |
| Target sequence: 5'- AAGCAGTGCGTTAACTATCTA -3' (SEQ ID NO: 1) |
| Sense sequence: 5'- r(GCAGUGCGUUAACUAUCUA)dTdT -3' |
| Antisense sequence: 5'- r(UAGAUAGUUAACGCACUGC)dTdT -3' |

**[Table 2]**

| |
|---|
| Cat. no.: SI02658999 |
| Product Name: Hs_CTDSP2_6 HP GenomeWide siRNA |
| Target sequence: 5'- TACGATCAGCGTGACAGAGTA -3' (SEQ ID NO: 2) |
| Sense sequence: 5'- r(CGAUCAGCGUGACAGAGUA)dTdT -3' |
| Antisense sequence: 5'- r(UACUCUGUCACGCUGAUCG)dTdA -3' |

**[Table 3]**

| |
|---|
| Cat. no.: SI03100048 |
| Product Name: Hs_DUSP1_5 HP GenomeWide siRNA |
| Target sequence: 5'- CTGGTTCAACGAGGCCATTGA -3' (SEQ ID NO: 3) |
| Sense sequence: 5'- r(GGUUCAACGAGGCCAUUGA)dTdT -3' |
| Antisense sequence: 5'- r(UCAAUGGCCUCGUUGAACC)dAdG -3' |

**[Table 4]**

| |
|---|
| Cat. no. : SI00447398 |
| Product Name: Hs_IMPA2_3 HP GenomeWide siRNA |
| Target sequence: 5'- CTGCAGATCTTGTGACAGAAA -3' (SEQ ID NO: 4) |
| Sense sequence: 5'- r(GCAGAUCUUGUGACAGAAA)dTdT -3' |
| Antisense sequence: 5'- r(UUUCUGUCACAAGAUCUGC)dAdG -3' |

**[Table 5]**

| |
|---|
| Cat. no.: SI00702800 |
| Product Name: Hs_RHOBTB3_4 HP GenomeWide siRNA |
| Target sequence: 5'- AAGCCTTAAATCAGAAGACAA -3' (SEQ ID NO: 5) |
| Sense sequence: 5'- r(GCCUUAAAUCAGAAGACAA)dTdT -3' |
| Antisense sequence: 5'- r(UUGUCUUCUGAUUUAAGGC)dTdT -3' |

**[Table 6]**

| |
|---|
| Cat. no.: SI00287798 |
| Product Name: Hs_SGK_5 HP GenomeWide siRNA |
| Target sequence: 5'- CACAGCTGAAATGTACGACAA -3' (SEQ ID NO: 6) |
| Sense sequence: 5'- r(CAGCUGAAAUGUACGACAA)dTdT -3' |
| Antisense sequence: 5'- r(UUGUCGUACAUUUCAGCUG)dTdG -3' |

**[Table 7]**

| |
|---|
| Cat. no.: SI02651243 |
| Product Name: Hs_UBE2H_4 HP GenomeWide siRNA |
| Target sequence: 5'- AAGGCGGAGTATGGAAAGTTA -3' (SEQ ID NO: 7) |
| Sense sequence: 5'- r(GGCGGAGUAUGGAAAGUUA)dTdT -3' |
| Antisense sequence: 5'- r(UAACUUUCCAUACUCCGCC)dTdT -3' |

**[Table 8]**

| |
|---|
| Cat. no. : SI02659447 |
| Product Name: Hs_INPP5F_6_HP Validated siRNA |
| Target sequence: 5' - CAGATCTTCCATGGTGGCTTA - 3' (SEQ ID NO: 8) |

| |
|---|
| Sense sequence: 5' - r(GAUCUUCCAUGGUGGCUUA)dTdT - 3' |
| Antisense sequence: 5' - r(UAAGCCACCAUGGAAGAUC)dTdG - 3' |

**[Table 9]**

| |
|---|
| Cat. no.: SI02223004 |
| Product Name: Hs_MAP2K6_6_HP Validated siRNA |
| Target sequence: 5' - TAGACCTATGATAAATAACCA - 3' (SEQ ID NO: 9) |
| Sense sequence: 5' - r(GACCUAUGAUAAAUAACCA)dTdT - 3' |
| Antisense sequence: 5' - r(UGGUUAUUUAUCAUAGGUC)dTdA - 3' |

**[Table 10]**

| |
|---|
| Cat. no.: SI02659146 |
| Product Name: Hs_PPMIE_8_HP Validated siRNA |
| Target sequence: 5' - GAGGCGGTTTATAGTCAGAAA - 3' (SEQ ID NO: 10) |
| Sense sequence: 5' - r(GGCGGUUUAUAGUCAGAAA)dTdT - 3' |
| Antisense sequence: 5' - r(UUUCUGACUAUAAACCGCC)dTdC - 3' |

**[Table 11]**

| |
|---|
| Cat. no. : SI02759043 |
| Product Name: Hs_PRKAG2_6_HP Validated siRNA |
| Target sequence: 5' - AACATTTAAGCCTTTAGTGAA - 3' (SEQ ID NO: 11) |
| Sense sequence: 5' - r(CAUUUAAGCCUUUAGUGAA)dTdT- 3' |
| Antisense sequence: 5' - r(UUCACUAAAGGCUUAAAUG)dTdT - 3' |

**[Table 12]**

| |
|---|
| Cat. no. : SI00301329 |
| Product Name: Hs_PRKCD_7_HP Validated siRNA |
| Target sequence: 5' - AACTCTACCGTGCCACGTTTT - 3' (SEQ ID NO: 12) |
| Sense sequence: 5' - r(CUCUACCGUGCCACGUUUU)dTdT - 3' |
| Antisense sequence: 5' - r(AAAACGUGGCACGGUAGAG)dTdT- 3' |

**[Table 13]**

| |
|---|
| Cat. no. : SI02659076 |
| Product Name: Hs_PTPN21_7_HP Validated siRNA |
| Target sequence: 5' - GAGGAGACCATTCAATTTCAA - 3' (SEQ ID NO: 13) |
| Sense sequence: 5' - r(GGAGACCAUUCAAUUUCAA)dTdT- 3' |
| Antisense sequence: 5' - r(UUGAAAUUGAAUGGUCUCC)dTdC- 3' |

**[Table 14]**

| |
|---|
| Cat. no. : SI03103863 |
| Product Name: Hs_UBE2B_7_HP GenomeWide siRNA |
| Target sequence: 5' - GAGGCTCATGCGGGATTTCAA - 3' (SEQ ID NO: 14) |
| Sense sequence: 5' - r(GGCUCAUGCGGGAUUUCAA)dTdT - 3' |
| Antisense sequence: 5' - r(UUGAAAUCCCGCAUGAGCC)dTdC - 3' |

**[Table 15]**

| |
|---|
| Cat. no. : SI00754978 |
| Product Name: Hs_UBTF_2_HP GenomeWide siRNA |
| Target sequence: 5' - CAGGACTTCCAGAGAGAGAAA - 3' (SEQ ID NO: 15) |
| Sense sequence: 5' - r(GGACUUCCAGAGAGAGAAA)dTdT- 3' |
| Antisense sequence: 5' - r(UUUCUCUCUCUGGAAGUCC)dTdG- 3' |

**[Table 16]**

| |
|---|
| Cat. no. : SI03147886 |
| Product Name: Hs_ZNF259_5_HP GenomeWide siRNA |
| Target sequence: 5' - AGGTTATTTATTAGTATTGGA - 3' (SEQ ID NO: 16) |
| Sense sequence: 5' - r(GUUAUUUAUUAGUAUUGGA)dTdT - 3' |
| Antisense sequence: 5' - r(UCCAAUACUAAUAAAUAAC)dTdT- 3' |

In an *in vitro* cell culture system, incorporation of a prepared expression-inhibiting substance (e.g., antisense nucleotide, ribozyme, or siRNA) into cancer cells of interest may be carried out through, for example, electroporation, lipofection, viral infection employing a virus vector (e.g., adenovirus or retrovirus), or transfection employing calcium.
In the case where an expression inhibitor containing, as an active ingredient, the above-prepared expression-inhibiting substance (e.g., antisense nucleotide, ribozyme, or siRNA) is incorporated into an individual (e.g., human or a vertebrate other than human) *in vivo*, the expression inhibitor may be incorporated directly into a region in the vicinity of cancer cells of interest, or may be administered through an oral, intradermal, subcutaneous, intravenous, intramuscular, or intraperitoneal route. For systemic administration of the expression inhibitor, transmucosal or transdermal administration may be carried out by use of a penetrant such as a bile salt, fuchsin acid, or a surfactant. Such a pharmaceutical composition may be topically administered, or may be administered in the form of, for example, plaster, paste, or gel.
For administration of the expression inhibitor to an individual, the expression inhibitor is preferably prepared in such a form that it is readily incorporated into cells. For example, appropriate cells may be infected *in vitro* with an expression vector prepared by incorporating, into a virus vector, a DNA fragment encoding the aforementioned antisense nucleotide, ribozyme, or siRNA for production of a virus, and then an individual may be infected with the virus through injection. The virus vector employed may be an intracellularly expressible adenovirus vector or retrovirus vector.
The aforementioned expression vector may be inserted into liposomes and the liposomes may be fused with cancer cells for intracellular incorporation of the plasmid.
Alternatively, there may be injected, as the expression inhibitor, an RNA aptamer prepared by binding, through the *in vitro* selection method, the above-prepared RNA (e.g., antisense nucleotide, ribozyme, or siRNA) to a peptide which is readily incorporated into cells (e.g., TAT of HIV).

In the present invention, no particular limitation is imposed on the ABC transporter protein expression inhibitor, so long as it exhibits the effect of inhibiting expression of an ABC transporter protein. However, the ABC transporter protein expression inhibitor preferably attains a percent reduction in expression of P-glycoprotein, which is on the basis of relative fluorescence intensity (channel) determined through the method described hereinbelow (Example 1), of 22 to 83%, more preferably 22 to 50%, particularly preferably 22 to 40%, most preferably 22 to 30%.
The ABC transporter protein expression inhibitor preferably attains a percent reduction in expression of BCRP, which is on the basis of relative fluorescence intensity (channel) determined through the method described hereinbelow (Example 2), of 37 to 87%, more preferably 37 to 60%, particularly preferably 37 to 50%, most preferably 37 to 40%.

Through the aforementioned method, expression inhibition by an expression-inhibiting substance may be evaluated by comparing, in the amount of mRNA transcribed from a gene of interest whose expression is to be inhibited, between *in vitro*-cultured specific cancer cells into which the expression-inhibiting substance has been incorporated, and those in which the expression-inhibiting substance has not been incorporated. The amount of mRNA may be determined through, for example, RT-PCR or northern blotting. On the basis of the thus-obtained results, an ABC transporter protein expression inhibitor may be selected through screening of substances which can more effectively inhibit translation of mRNA transcribed from the gene of interest whose expression is to be inhibited. Specifically, preferably, there is employed a method in which human breast cancer cells MCF-7 are treated with a test substance, and then mRNA is purified from collected cells, followed by Affimetryx DNA microarray analysis for evaluation of change in expression of the gene of interest. No particular limitation is imposed on the cells which are preferably employed in the aforementioned screening method, so long as one or more genes targeted by the ABC transporter protein expression inhibitor of the present invention are expressed in the cells.

Similar to the case of the aforementioned *in vitro* experiment, expression inhibition by an expression-inhibiting substance may be evaluated in an *in vivo* experiment. That is, expression inhibition by an expression inhibitor may be evaluated by administering the expression inhibitor to a non-human animal with cancer, and comparing the cancer size and survival rate of the animal with those of a non-human animal with cancer to which the expression inhibitor has not been administered. Specifically, expression inhibition by an expression-inhibiting substance may be evaluated through the following procedure: the aforementioned specific cancer cells are administered to a normal mouse; tumor is enlarged for a certain period of time; subsequently, the above-prepared expression inhibitor is administered once to several times through the aforementioned incorporation method; and then the cancer size and survival rate of the mouse are compared with those of a mouse to which the expression inhibitor has not been administered.

In the present invention, when the expression level of a gene of interest is determined in a test sample (e.g., cancer cells (tissue) excised from a patient, or a biopsy sample), and the sensitivity of the cancer cells to an anticancer drug is determined by comparing the expression level with a specific level (e.g., the expression level of the gene in normal cancer cells which do not exhibit resistance to the anticancer drug), the anticancer drug resistance of the test cancer cells can be determined. In the case where the expression level of the gene of interest in cancer cells of the subject is lower than a specific expression level, the cancer cells are determined to be sensitive to the anticancer drug (i.e., the cancer cells are determined to exhibit low anticancer drug resistance). In the case where the expression level of the gene of interest in cancer cells of the subject is higher than the specific expression level, the cancer cells are determined to be less sensitive to the anticancer drug (i.e., the cancer cells are determined to exhibit high anticancer drug resistance). When cancer cells are less sensitive to an anticancer drug, the effect of the drug on the cancer cells are not expected, and the drug may only cause side effects. Thus, the determination method of the present invention can prevent occurrence of undesirable side effects, or progress of cancer or increase in side effects associated with continuation of ineffective treatment.
According to the below-described method, the degree of side effects which may occur after administration of an anticancer drug can be predicted more correctly. Specifically, when the expression level of a gene of interest is determined in test cells (e.g., normal cells from a subject), and the sensitivity of the normal cells to an anticancer drug is determined by comparing the expression level with a specific level (e.g., the expression level of the gene in normal cells of a healthy subject), the degree of side effects which may occur after administration of the anticancer drug can be predicted, which may lead to safe drug administration. In the case where the expression level of the gene of interest in normal cells of the subject is lower than a specific expression level, the subject is determined to be sensitive to the anticancer drug (i.e., the probability of occurrence of side effects which may occur after administration of the anticancer drug is high). In the case where the expression level of the gene of interest in normal cells of the subject is higher than the specific expression level, the subject is determined to be less sensitive to the anticancer drug (i.e., the probability of occurrence of side effects which may occur after administration of the anticancer drug is low).

No particular limitation is imposed on the anticancer drug targeted by the expression inhibitor of the present invention, so long as cancer cells acquire resistance thereto by the mediation of an ABC transporter protein such as BCRP or P-glycoprotein. Examples of the anticancer drug include camptothecins such as irinotecan hydrochloride, topotecan, and topotecin; anthraquinones such as mitoxantrone; staurosporines such as 7-hydroxystaurosporine; anthracyclines such as doxorubicin hydrochloride, daunomycin, epirubicin hydrochloride, and adriamycin; vinca alkaloids such as vincristine; taxanes such as paclitaxel and docetaxel; etoposide; mitomycin; gefitinib; imatinib; and erlotinib.

No particular limitation is imposed on the cancer targeted by the method for determining sensitivity to an anticancer drug of the present invention, the method for predicting the degree of side effects which may occur after administration of an anticancer drug of the present invention, the method for determining anticancer drug resistance of the present invention, or the anticancer-drug-resistance-overcoming agent of the present invention, so long as the cancer is a cancer to which any of the aforementioned anticancer drugs is applied.

Since the ABC transporter protein expression inhibitor of the present invention strongly inhibits expression of an ABC transporter protein, the inhibitor may be employed as an anticancer-drug-resistance-overcoming agent or an anticancer-drug-effect-enhancing agent. Specifically, the ABC transporter protein expression inhibitor may be employed as an anticancer-drug-resistance-overcoming agent for a cancer which has acquired ABC-transporter-associated resistance through administration of an anticancer drug, or may be employed as an anticancer-drug-effect-enhancing agent for a cancer which originally expresses an ABC transporter protein and exhibits low sensitivity to an anticancer drug.

When the anticancer-drug-resistance-overcoming agent (A) of the present invention is employed in combination with any of the aforementioned anticancer drugs (B) to which cancer cells can acquire resistance, the therapeutic effect on a cancer which has acquired anticancer drug resistance is recovered. Therefore, a combination of these ingredients (A) and (B) is useful as a new anticancer pharmaceutical composition.

The expression inhibitor or new anticancer drug of the present invention may be administered in such a way that conventional drug products, each containing the above ingredients, may be administered in combination. Alternatively, a new drug product containing the above ingredients may be provided. Examples of the form of such a drug product include an oral product, an injection (for intramuscular, subcutaneous, or intravenous injection), a suppository, and an external-use agent (for patch or application).

No strict limitation is imposed on the dose of the ABC transporter protein expression inhibitor of the present invention. However, preferably, the dose of the ABC transporter protein expression inhibitor is appropriately determined after, for example, a pharmacokinetic test, since the inhibitor exhibits different effects under different conditions of use thereof (e.g., a subject or a disease to which the inhibitor is applied). When, for example, the ABC transporter protein expression inhibitor employs siRNA, the daily dose of the inhibitor for an adult is generally 0.01 µg/kg to 10 mg/kg. The dose of an anticancer drug (B) to which cancer cells can acquire resistance may be a general effective amount. For example, the daily dose of the anticancer drug for an adult is generally 1 mg to 1 g, particularly preferably 2 mg to 300 mg.

ABC transporter protein expression inhibitors of the present invention may be employed singly or in combination of a plurality of species. The ABC transporter protein expression inhibitor(s) may be employed in combination with an additional compound which provides therapeutic advantages. The mechanism of action of the additional compound may be identical to or different from that of the compound of the present invention.

The drug of the present invention may be provided in the form of, for example, a solid product (e.g., tablet, granules, powder, or capsule), a liquid product (e.g., solution, suspension, or emulsion), or a lyophilized product. Such a drug product may be prepared through a customary technique for drug production by use of a pharmaceutically acceptable carrier. Examples of the aforementioned pharmaceutically acceptable carrier include starch, dextrin, fatty acid glyceride, polyethylene glycol, hydroxyethyl starch, ethylene glycol, polyoxyethylene sorbitan fatty acid ester, amino acid, gelatin, albumin, water, and saline. If necessary, the drug product may appropriately contain a conventional additive such as a stabilizer, a humectant, an emulsifier, a binder, an isotonic agent, or an excipient.

The ABC transporter protein expression inhibitor of the present invention may be employed not only in the form of the aforementioned drug product, but also in the form of, for example, a food or beverage. When the ABC transporter protein expression inhibitor of the present invention is incorporated into a food or beverage, the inhibitor may be employed as is, or mixed with various nutritional ingredients. Specifically, when the ABC transporter protein expression inhibitor of the present invention is incorporated into a food or beverage, the inhibitor may be appropriately mixed with an additive which can be used in a food or beverage, and the mixture may be prepared, through conventional means, into a form suitable for edible use; for example, granules, particles, tablet, capsule, or paste. The inhibitor may be added to a variety of foods; for example, processed meat products (e.g., ham and sausage), processed fish products (e.g., kamaboko and *chikuwa),* bread, confectionary, butter, powdered milk, and fermented foods and beverages. Alternatively, the inhibitor may be added to beverages such as water, fruit juice, milk, refreshing beverages, and tea beverages. As used herein, the term "food or beverage" encompasses animal feeds.

Examples of preferred foods and beverages include fermented dairy products containing the ABC transporter protein expression inhibitor of the present invention, such as fermented milk, lactic acid bacteria beverages, fermented soybean milk, fermented fruit juice, and fermented plant extract. Such a fermented dairy food or beverage may be produced through a customary method. For example, a fermented milk product may be produced through the following procedure. Firstly, lactic acid bacteria or bifidobacteria are inoculated into a sterilized milk medium, followed by culturing, and the cultured product is homogenized to thereby produce a fermented milk base. Subsequently, a separately prepared syrup and the ABC transporter protein expression inhibitor of the present invention are added to and mixed with the fermented milk base, and the mixture is homogenized by means of, for example, a homogenizer, followed by addition of a flavor to the resultant mixture, to thereby produce a final product. The thus-produced fermented milk product may be provided in any form, such as a plain-type product, a soft-type product, a fruit-flavor-type product, a solid product, or a liquid product.

The ABC transporter protein expression inhibitor of the present invention can be applied to all mammals (including human).

### Examples

The present invention will next be described in more detail by way of examples, which should not be construed as limiting the invention thereto.

### Example 1 Change in expression of P-glycoprotein through transfection of siRNA

### (1) Test method

Next will be described a method for determining change in expression of P-glycoprotein through transfection of siRNA.
Human breast cancer cells expressing exogenous P-glycoprotein (MCF-7/MDR) (the same cells as MCF-7/MDR1 described in JP-A-2006-69910) were inoculated (4 × 10⁵ cells) into 60 mm-dish (product of IWAKI) and cultured for 16 hours. Commercially available siRNA (product of Qiagen) (shown in Tables 1 to 16), which targets a gene of interest and has been shown to inhibit expression of the gene, was transfected into the thus-cultured cells. Specifically, a 20 µmol/L siRNA solution (4 µL, siRNA: 80 pmol) and OPTI-MEM (product of Gibco) (196 µL) were added to a microtube. They were mixed together through pipetting five times by means of a micropipette, and the mixture was allowed to stand still at room temperature for five minutes. Lipofectamine 2000 (product of Invitrogen) (6 µL) and OPTI-MEM (194 µL) were added to another microtube, and they were mixed together through pipetting five times by means of a micropipette. The entire Lipofectamine 2000/OPTI-MEM mixture was added to the siRNA/OPTI-MEM mixture which had been allowed to stand still for five minutes (total amount: 400 µL), followed by mixing through pipetting five times by means of a micropipette. The resultant siRNA/Lipofectamine 2000/OPTI-MEM mixture was allowed to stand still at room temperature for 20 minutes. During this mixing process, the MCF-7/MDR cells were washed with PBS(-) (product of Nissui Pharmaceutical Co., Ltd.) (4 mL), and DMEM medium (product of Sigma) (1.6 mL) containing ampicillin (product of Sigma) (final concentration: 50 µg/mL) was added to the cells. After the siRNA/Lipofectamine 2000/OPTI-MEM mixture had been allowed to stand still at room temperature for 20 minutes, the entire mixture (400 µL) was added to the MCF-7/MDR cells, followed by gentle shaking. After determination of formation of a uniform mixture, culturing was carried out at 37°C and 5% CO₂ for 72 hours.

The expression level of P-glycoprotein on the cell surfaces was determined through FACS (fluorescence activated cell sorting). For determination of the expression level through FACS, P-glycoprotein which would be expressed on the cell surfaces was stained with a labeled antibody, and the thus-stained cells were exposed to fluid flow, to thereby measure the amount of labeled molecules (P-glycoprotein). Specifically, 5 × 10⁵ cells were suspended in 10% Gammagard/Hanks buffer (product of Nissui Pharmaceutical Co., Ltd.) (200 µL) and allowed to stand still on ice for 15 minutes, to thereby block the proteins on the cell surfaces. Subsequently, P-glycoprotein expressed on the cell surfaces was reacted with biotinylated P-glycoprotein antibody (MRK16) (final concentration: 100 µg/mL)/10% Gammagard/Hanks buffer (50 µL), and then reacted with 40% PE-labeled streptavidin (product of Becton, Dickinson and Company)/10% Gammagard/Hanks buffer (50 µL). Thereafter, the intensity of PE (relative fluorescence intensity: channel) was measured through FACS, to thereby determine the amount of P-glycoprotein expressed on the cell surfaces.

### (2) Results

Table 17 shows change in expression level of P-glycoprotein through transfection of siRNA shown in Tables 1 to 16. Change in expression level of P-glycoprotein was determined on the basis of "b/a × 100(%)," wherein "a" represents the median of relative fluorescence intensities (channel) in untreated cells (i.e., cells into which siRNA was not transfected), and "b" represents the median of relative fluorescence intensities (channel) in cells after transfection of siRNA thereinto.

As shown in Table 17, when siRNA shown in Tables 1 to 16 was employed, the expression level of P-glycoprotein (relative fluorescence intensity (channel)) was reduced to 22 to 83% of that determined in untreated cells.
Thus, when expression of the following gene: ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, or ZNF259 is inhibited, the expression level of P-glycoprotein is considerably reduced. Therefore, a substance which inhibits expression of any of these genes (e.g., siRNA shown in Tables 1 to 16) is suitable for use as a P-glycoprotein expression inhibitor.

**[Table 17]**

| Change in expression of P-glycoprotein through transfection of siRNA | | | | |
|---|---|---|---|---|
| Table No. of siRNA | Gene of interest | a: relative fluorescence intensity (channel) in untreated cells | b: relative fluorescence intensity (channel) in cells after transfection | b/a×100(%) |
| 1 | ARHGAP17 | 328 | 178 | 54 |
| 2 | CTDSP2 | 316 | 165 | 52 |
| 3 | DUSP1 | 365 | 120 | 33 |
| 4 | IMPA2 | 316 | 129 | 41 |
| 5 | RHOBTB3 | 365 | 93 | 25 |
| 6 | SGK | 365 | 165 | 45 |
| 7 | UBE2H | 365 | 81 | 22 |
| 8 | INPP5F | 349 | 207 | 59 |
| 9 | MAP2K6 | 567 | 211 | 37 |
| 10 | PPM1E | 461 | 279 | 61 |
| 11 | PRKAG2 | 461 | 250 | 54 |
| 12 | PRKCD | 461 | 382 | 83 |
| 13 | PTPN21 | 461 | 227 | 49 |
| 14 | UBE2B | 246 | 171 | 70 |
| 15 | UBTF | 557 | 262 | 47 |
| 16 | ZNF259 | 557 | 302 | 54 |

### Example 2 Change in expression of BCRP through transfection of siRNA

### (1) Test method

Next will be described a method for determining change in expression of BCRP through transfection of siRNA.
Human breast cancer cells expressing exogenous BCRP (MCF-7/BCRP) (JP-A-2003-63989) were inoculated (4 × 10⁵ cells) into 60 mm-dish (product of IWAKI) and cultured for 16 hours. Commercially available siRNA (product of Qiagen) (shown in Tables 1 to 16), which targets a gene of interest and has been shown to inhibit expression of the gene, was transfected into the thus-cultured cells. Specifically, a 20 µmol/L siRNA solution (4 µL, siRNA: 80 pmol) and OPTI-MEM (product of Gibco) (196 µL) were added to a microtube. They were mixed together through pipetting five times by means of a micropipette, and the mixture was allowed to stand still at room temperature for five minutes. Lipofectamine 2000 (product of Invitrogen) (6 µL) and OPTI-MEM (194 µL) were added to another microtube, and they were mixed together through pipetting five times by means of a micropipette. The entire Lipofectamine 2000/OPTI-MEM mixture was added to the siRNA/OPTI-MEM mixture which had been allowed to stand still for five minutes (total amount: 400 µL), followed by mixing through pipetting five times by means of a micropipette. The resultant siRNA/Lipofectamine 2000/OPTI-MEM mixture was allowed to stand still at room temperature for 20 minutes. During this mixing process, the MCF-7/BCRP cells were washed with PBS(-) (product of Nissui Pharmaceutical Co., Ltd.) (4 mL), and DMEM medium (product of Sigma) (1.6 mL) containing ampicillin (product of Sigma) (final concentration: 50 µg/mL) was added to the cells. After the siRNA/Lipofectamine 2000/OPTI-MEM mixture had been allowed to stand still at room temperature for 20 minutes, the entire mixture (400 µL) was added to the MCF-7/BCRP cells, followed by gentle shaking. After determination of formation of a uniform mixture, culturing was carried out at 37°C and 5% CO₂ for 72 hours.

The expression level of BCRP on the cell surfaces was determined through FACS (fluorescence activated cell sorting). For determination of the expression level through FACS, BCRP which would be expressed on the cell surfaces was stained with a labeled antibody, and the thus-stained cells were exposed to fluid flow, to thereby measure the amount of labeled molecules (BCRP). Specifically, 5 × 10⁵ cells were suspended in 10% Gammagard/Hanks buffer (product of Nissui Pharmaceutical Co., Ltd.) (200 µL) and allowed to stand still on ice for 15 minutes, to thereby block the proteins on the cell surfaces. Subsequently, BCRP expressed on the cell surfaces was reacted with biotinylated BCRP antibody (anti BCRP-Biotin) (final concentration: 100 µg/mL)/10% Gammagard/Hanks buffer (50 µL), and then reacted with 40% PE-labeled streptavidin (product of Becton, Dickinson and Company)/10% Gammagard/Hanks buffer (50 µL). Thereafter, the intensity of PE (relative fluorescence intensity: channel) was measured through FACS, to thereby determine the amount of BCRP expressed on the cell surfaces.

### (2) Results

Table 18 shows change in expression level of BCRP through transfection of siRNA shown in Tables 1 to 16. Change in expression level of BCRP was determined on the basis of "b/a × 100(%)," wherein "a" represents the median of relative fluorescence intensities (channel) in untreated cells (i.e., cells into which siRNA was not transfected), and "b" represents the median of relative fluorescence intensities (channel) in cells after transfection of siRNA thereinto.

As shown in Table 18, when siRNA shown in Tables 1 to 16 was employed, the expression level of BCRP (relative fluorescence intensity (channel)) was reduced to about 37 to about 87% of that determined in untreated cells.
Thus, when expression of the following gene: ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, or ZNF259 is inhibited, the expression level of BCRP is considerably reduced. Therefore, a substance which inhibits expression of any of these genes (e.g., siRNA shown in Tables 1 to 16) is suitable for use as a BCRP expression inhibitor.

**[Table 18]**

| Change in expression of BCRP through transfection of siRNA | | | | |
|---|---|---|---|---|
| Table No. of siRNA | Gene of interest | a: relative fluorescence intensity (channel) in untreated cells | b: relative fluorescence intensity (channel) in cells after transfection | b/a×100(%) |
| 1 | ARHGAP17 | 189 | 103 | 54 |
| 2 | CTDSP2 | 189 | 81 | 43 |
| 3 | DUSP1 | 189 | 99 | 52 |
| 4 | IMPA2 | 189 | 78 | 41 |
| 5 | RHOBTB3 | 189 | 150 | 79 |
| 6 | SGK | 189 | 89 | 47 |
| 7 | UBE2H | 189 | 71 | 38 |
| 8 | INPP5F | 302 | 160 | 53 |
| 9 | MAP2K6 | 150 | 76 | 51 |
| 10 | PPM1E | 189 | 87 | 46 |
| 11 | PRKAG2 | 189 | 69 | 37 |
| 12 | PRKCD | 189 | 95 | 50 |
| 13 | PTPN21 | 211 | 126 | 60 |
| 14 | UBE2B | 189 | 165 | 87 |
| 15 | UBTF | 211 | 97 | 46 |
| 16 | ZNF259 | 211 | 143 | 68 |

## Claims

1. An ABC transporter protein expression inhibitor containing, as an active ingredient, a substance which inhibits expression of one or more genes selected from the group consisting of ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, and ZNF259.

2. An ABC transporter protein expression inhibitor according to claim 1, wherein the substance which inhibits expression of one or more genes selected from the group consisting of ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, and ZNF259 is siRNA.

3. An anticancer-drug-resistance-overcoming agent for cancer cells which have acquired anticancer drug resistance by the mediation of an ABC transporter protein, the agent containing, as an active ingredient, the substance as recited in claim 1 or 2.

4. A pharmaceutical composition comprising the substance as recited in claim 1 or 2 in combination with an anticancer drug capable of serving as a substrate for an ABC transporter protein.

5. Use of the substance as recited in claim 1 or 2 for producing an ABC transporter protein expression inhibitor, or an anticancer-drug-resistance-overcoming agent for cancer cells which have acquired anticancer drug resistance by the mediation of an ABC transporter protein.

6. Use, for producing a pharmaceutical composition, of the substance as recited in claim 1 or 2 and an anticancer drug in combination, wherein the anticancer drug is capable of serving as a substrate for an ABC transporter protein.

7. A method for inhibiting expression of an ABC transporter protein, or a method for overcoming the anticancer drug resistance of cancer cells that has been acquired by the mediation of an ABC transporter protein, the method comprising administering an effective amount of the substance as recited in claim 1 or 2 to a subject in need thereof.

8. A method for treating cancer, the method comprising administering, to a subject in need thereof, the substance as recited in claim 1 or 2, and an anticancer drug capable of serving as a substrate for an ABC transporter protein.

9. A screening method for selecting an ABC transporter protein expression inhibitor, the method comprising searching a substance which inhibits expression of one or more genes selected from the group consisting of ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, and ZNF259.

10. A method for determining sensitivity to an anticancer drug capable of serving as a substrate for an ABC transporter protein, the method comprising measuring the expression level of one or more genes selected from the group consisting of ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, and ZNF259.

11. A method for predicting the degree of side effects which may occur after administration of an anticancer drug capable of serving as a substrate for an ABC transporter protein, the method comprising measuring the expression level of one or more genes selected from the group consisting of ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, and ZNF259.

12. A method for determining anticancer drug resistance by the mediation of an ABC transporter protein, the method comprising measuring the expression level of one or more genes selected from the group consisting of ARHGAP17, CTDSP2, DUSP1, IMPA2, RHOBTB3, SGK, UBE2H, INPP5F, MAP2K6, PPM1E, PRKAG2, PRKCD, PTPN21, UBE2B, UBTF, and ZNF259.


SEQUENCE LISTING

<110>  Sugimoto Yoshikazu

<120>  ABC transporter protein expressin inhibitor

<130>  YK0046

<150>  JP 2008-090729
<151>  2008-03-31

<160>  16

<170>  PatentIn version 3.1

<210>  1
<211>  21
<212>  DNA
<213>  Artificial Sequence

<220>
<223>  Target sequence of  ARHGAP17 gene

<400>  1
aagcagtgcg ttaactatct a                                                21

<210>  2
<211>  21
<212>  DNA
<213>  Artificial Sequence

<220>
<223>  Target sequence of CTDSP2 gene

<400>  2
tacgatcagc gtgacagagt a                                                21

<210>  3
<211>  21
<212>  DNA
<213>  Artificial Sequence

<220>
<223>  Target sequence of DUSP1 gene

<400>  3
ctggttcaac gaggccattg a                                                21

<210>  4
<211>  21
<212>  DNA
<213>  Artificial Sequence

<220>
<223>  Target sequence of IMPA2 gene

<400>  4
ctgcagatct tgtgacagaa a                                                21

<210>  5
<211>  21
<212>  DNA
<213>  Artificial Sequence

<220>
<223>  Target sequence of RHOBTB3 gene

<400> 5
aagccttaaa tcagaagaca a 21

<210> 6
<211> 21
<212> DNA
<213> Artificial Sequence

<220>
<223> Target sequence of SGK gene

<400> 6
cacagctgaa atgtacgaca a 21

<210> 7
<211> 21
<212> DNA
<213> Artificial Sequence

<220>
<223> Target sequence of UBE2H gene

<400> 7
aaggcggagt atggaaagtt a 21

<210> 8
<211> 21
<212> DNA
<213> Artificial Sequence

<220>
<223> Target sequence of INPP5F gene

<400> 8
cagatcttcc atggtggctt a 21

<210> 9
<211> 21
<212> DNA
<213> Artificial Sequence

<220>
<223> Target sequence of MAP2K6 gene

<400> 9
tagacctatg ataaataacc a 21

<210> 10
<211> 21
<212> DNA
<213> Artificial Sequence

<220>
<223> Target sequence of PPM1E gene

<400> 10
gaggcggttt atagtcagaa a 21

<210> 11
<211> 21
<212> DNA
<213> Artificial Sequence

<220>
<223>  Target sequence of PRKAG2 gene

<400>  11
aacatttaag cctttagtga a                                                21


<210>  12
<211>  21
<212>  DNA
<213>  Artificial Sequence

<220>
<223>  Target sequence of PRKCD gene

<400>  12
aactctaccg tgccacgttt t                                                21


<210>  13
<211>  21
<212>  DNA
<213>  Artificial Sequence

<220>
<223>  Target sequence of PTPN21 gene

<400>  13
gaggagacca ttcaatttca a                                                21


<210>  14
<211>  21
<212>  DNA
<213>  Artificial Sequence

<220>
<223>  Target sequence of UBE2B gene

<400>  14
gaggctcatg cgggatttca a                                                21


<210>  15
<211>  21
<212>  DNA
<213>  Artificial Sequence

<220>
<223>  Target sequence of UBTF gene

<400>  15
caggacttcc agagagagaa a                                                21


<210>  16
<211>  21
<212>  DNA
<213>  Artificial Sequence

<220>
<223>  Target sequence of ZNF259 gene

<400>  16
aggttattta ttagtattgg a                                                21